(19) Europäisches Patentamt
European Patent Office
Office européen des brevets

(11) EP 1 174 122 B1

(12) # FASCICULE DE BREVET EUROPEEN

(45) Date de publication et mention de la délivrance du brevet:
**13.06.2012 Bulletin 2012/24**

(21) Numéro de dépôt: **01401836.0**

(22) Date de dépôt: **09.07.2001**

(51) Int Cl.:
***A61K 8/02*** (2006.01) ***A61K 8/36*** (2006.01)
***A61K 8/44*** (2006.01) ***A61K 8/46*** (2006.01)
***A61K 8/60*** (2006.01) ***A61K 8/25*** (2006.01)
***A61K 8/55*** (2006.01) ***A61K 8/86*** (2006.01)
***A61Q 1/14*** (2006.01) ***A61Q 5/02*** (2006.01)
***A61Q 19/10*** (2006.01)

(54) **Composition cosmétique de nettoyage**

Kosmetisches Reinigungsmittel

Cleansing cosmetic composition

(84) Etats contractants désignés:
**AT BE CH CY DE DK ES FI FR GB GR IE IT LI LU MC NL PT SE TR**

(30) Priorité: **13.07.2000 FR 0009225**

(43) Date de publication de la demande:
**23.01.2002 Bulletin 2002/04**

(73) Titulaire: **L'Oréal**
**75008 Paris (FR)**

(72) Inventeurs:
- **Sebilotte-Arnaud, Laurence**
  **94240 L'Hay les Roses (FR)**
- **Guillou, Véronique**
  **92160 Antony (FR)**

(74) Mandataire: **Duvert, Sandra**
**L'Oréal**
**D.I.P.I.**
**River PLaza**
**25-29 Quai Aulagnier**
**92600 Asnieres-sur-Seine (FR)**

(56) Documents cités:
**EP-A- 0 514 760** **WO-A-93/08793**
**WO-A-96/28140** **WO-A-97/49381**
**WO-A-99/25313** **WO-A-99/38488**

- **DATABASE CAPLUS [en ligne] retrieved from STN Database accession no. 1987:38232 XP002168470 & JP 61 180712 A (KOBAYASHI KOSE CO.) 13 août 1986 (1986-08-13)**
- **DATABASE WPI Week 199222 Derwent Publications Ltd., London, GB; AN 1992-180847 XP002168471 & JP 04 120015 A (KANSAI KOSO KK)**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 174 122 B1

## Description

La présente invention a trait à une composition de nettoyage moussante rinçable comprenant au moins une silice hydrophile et au moins un composé oxyalkyléné, et à l'utilisation de ladite composition notamment dans le domaine cosmétique comme produits de nettoyage ou de démaquillage de la peau, des yeux, du cuir chevelu et/ou des cheveux, ainsi que pour traiter les peaux grasses et/ou désinfecter la peau et/ou le cuir chevelu.

Le nettoyage de la peau est très important pour le soin du visage. Il doit être le plus performant possible car les résidus gras tels que l'excès de sébum, les restes des produits cosmétiques utilisés quotidiennement et les produits de maquillage s'accumulent dans les replis cutanés et peuvent obstruer les pores de la peau et entraîner l'apparition de boutons.

Un moyen pour bien nettoyer la peau est d'utiliser des produits de nettoyage moussants. Les produits de nettoyage moussants actuellement disponibles dans le commerce se présentent sous forme de pains, de gels ou de crèmes moussants, et ils peuvent ou non contenir des savons (sels d'acides gras). Les produits moussants avec savons ont l'avantage de donner une mousse onctueuse ; cependant, certains consommateurs reprochent à ces produits de provoquer des tiraillements dus à leur détergence trop importante. Pour avoir un produit mieux toléré, on cherche à diminuer le taux de savons. Toutefois, le produit a alors une viscosité insuffisante.

Par ailleurs, les produits moussants sans savons sont bien tolérés en général. Toutefois, ils se présentent généralement sous forme de produits liquides ou de gels relativement fluides. Afin d'épaissir les produits moussants sans savon, il est connu d'y rajouter des épaississants tels que des composés alkyl- ou acyloxyéthylénés, des polysaccharides comme les dérivés de cellulose, les gommes de guar et ses dérivés, des polymères acryliques. Toutefois, pour augmenter la viscosité d'un produit moussant sans savon et obtenir une texture épaisse, il est nécessaire d'introduire une grande quantité de ces épaississants. Or, lorsqu'on augmente le pourcentage des composés alkyl- ou acyl- oxyéthylénés, tels que les esters d'alkyl glucose oxyéthylénés comme par exemple le PEG-120 méthyl glucose dioléate ou encore le ceteareth-60 myristyl glycol qui sont classiquement utilisés, on obtient une composition qui n'est pas homogène et dont l'étalement sur la peau n'est pas homogène car elle s'étale par paquets, ce qui rend ces compositions rédhibitoires pour l'utilisateur. En outre, la mousse se développe alors difficilement. De plus, il n'est pas possible d'augmenter indéfiniment le pourcentage de ces épaississants, car ils ne sont alors plus solubles dans le milieu.

En outre, lorsqu'on augmente le pourcentage de gomme de cellulose, de gomme guar ou de polymère acrylique, on obtient un produit dont le démarrage en mousse est médiocre ou qui est difficile à rincer, du fait d'un dépôt filmogène donnant une sensation de peau mal nettoyée.

Ainsi, il subsiste le besoin de produits de nettoyage, moussants, rinçables et épais qui conservent néanmoins les propriétés requises pour des produits moussants, à savoir un bon mélange à l'eau, une transformation rapide en mousse et un bon rinçage, et une bonne tolérance, notamment dans le cas des produits moussants avec savon.

La demanderesse a découvert de manière surprenante que l'association de silice hydrophile et de composés oxyalkylénés (oxyéthyléné et/ou oxypropyléné) particuliers permettait l'obtention de produits nettoyants moussants épais, ne coulant pas et d'une viscosité appropriée à un produit de nettoyage crèmeux. Ces produits ont un caractère solide mou et rhéofluidifiant. On entend ici par « produit à caractère solide mou » un produit qui est malléable, qui ne s'écoule pas sous son propre poids, et qui a un angle de perte $\delta$ allant de 2 à 45° et un module complexe G* allant de $10^2$ à $10^5$ Pa pour des fréquences allant de 0,01 à 10 Hz, $\delta$ et G* étant définis ci après.

Certes, il est connu d'utiliser de la silice dans des compositions nettoyantes ou détergentes. Ainsi, par exemple, le document US-A-5,880,076 décrit une composition détergente liquide pouvant contenir des silices. Les documents EP-A-550 281 et US-A-5,389,279 citent la silice comme poudre pouvant être incorporée dans des compositions de nettoyage. Toutefois, les silices utilisées seules dans les produits moussants sans savon ne sont pas efficaces pour épaissir et obtenir la rhéologie recherchée. Les documents JP-A-61180712 et WO-A-9628140 décrivent d'associer une silice hydrophile et un composé oxyalkyléné pour épaissir des produits moussants.

Ainsi, la présente demande a pour objet une composition de nettoyage comprenant dans un milieu aqueux physiologiquement acceptable comportant au moins 35 % en poids d'eau par rapport au poids total de la composition, (1) au moins un tensioactif moussant, (2) au moins 1 % en poids d'au moins une silice hydrophile, par rapport au poids total de la composition, la silice hydrophile étant choisie parmi les silices d'origine pyrogénée ou d'origine précipitée, et la silice hydrophile ayant une surface spécifique de 30 à 500 $m^2/g$, une dimension moyenne de particules en nombre allant de 3 à 50 nm et une densité tassée allant de 40 à 200 g/l et (3) au moins un composé oxyalkyléné choisi parmi les dérivés alkyl ou acyl éthoxylés de polyol.

On entend ici par « milieu physiologiquement acceptable » un milieu compatible avec la peau, les muqueuses, le cuir chevelu, les yeux et/ou les cheveux. Par ailleurs, il s'agit d'un milieux aqueux, c'est-à-dire d'un milieu comportant une quantité d'eau d'au moins 35 % en poids, allant de préférence de 35 à 95 % en poids et mieux de 40 à 90 % en poids par rapport au poids total de la composition.

Les compositions de l'invention sont des compositions de nettoyage moussantes et rinçables, et elles se présentent sous forme de gel translucide, épais et se comportent comme un solide mou et rhéofluidifiant, c'est-à-dire

qu'elles ont un comportement viscoélastique, avec une valeur de module complexe G* allant de $10^2$ à $10^5$ Pa, et une valeur d'angle de perte $\delta$ allant de 2 et 45° pour des fréquences allant de $10^{-2}$ à 10 Hz.

G* et $\delta$ sont les paramètres viscoélatiques utilisés pour mesurer les propriétés physiques des fluides viscoélastiques comme expliqué dans « An introduction to rheology » de H.A. BARNES, J.F. HUTTON, K. WALTERS, pages 46 à 54, (Ed. Elsevier-1989).

G* est le module complexe (complex modulus) et $\delta$ est l'angle de perte (loss angle). G' et G" sont les composantes de $\overline{G^*}$. G' et G" sont respectivement le module de conservation (storage modulus) et le module de perte (loss modulus) et i est égal à $(-1)^{1/2}$. Les composantes G' et G" du module complexe sont obtenues à partir de la relation entre la contrainte sinusoïdale (oscillatory stress) et la déformation sinusoïdale (oscillatory strain).

Les mesures rhéologiques de G* et $\delta$ sont effectuées généralement en utilisant un Rhéomètre Haake RS150, à la température de 25°C, avec des corps de mesure de géométrie cône - plan, le diamètre du cône et la dimension du plan étant de 60 mm et l'angle du cône de 2° et l'entrefer entre le cône et le plan étant de 0,1 mm.

Pour faire des mesures dynamiques de viscoélasticité (oscillatory measurements), on détermine d'abord le domaine viscoélastique linéaire, en soumettant l'échantillon à des contraintes sinusoïdales d'amplitudes croissantes et de fréquence constante. Les modules sont reportés en fonction de l'amplitude de la contrainte (amplitude of stress) ou de l'amplitude de la déformation (strain) afin de déterminer les limites du domaine viscoélastique linéaire. Après avoir identifié le domaine viscoélastique linéaire, des mesures dynamiques sont réalisées dans la zone viscoélastique linéaire, pour une valeur de déformation constante située dans le domaine viscoélastique linéaire et à fréquence variable. Le rhéomètre Haake RS150 peut couvrir une gamme de fréquences variant de 0,01 to 10 Hz (soit 0,063 to 62,8 rad/sec).

A partir des valeurs des amplitudes de la contrainte $\tau_0$, de la déformation $\gamma_0$, ainsi qu'à partir du déphasage delta, on établit les relations suivantes :

$$G^* = \frac{\tau_0}{\gamma_0}$$

$$G' = G^*.\cos\delta$$

$$G'' = G^*.\sin\delta$$

$$\overline{G^*} = G' + iG''$$

Comme indiqué ci-dessus, le module G* de la composition de l'invention va de $10^2$ à $10^5$ Pa et $\delta$ varie de 2 à 45° pour une fréquence allant de $10^{-2}$ à 10 Hz.

Malgré la forte valeur de G* des compositions selon l'invention, la vitesse de démarrage de la mousse n'est pas diminuée et les qualités de mousse et de rinçage sont maintenues.

<u>Silices hydrophiles</u>

On entend par « silice hydrophile » dans la présente demande aussi bien les silices hydrophiles pures que les particules enrobées de silice hydrophile.

La quantité de silice(s) hydrophile(s) dans la composition de l'invention, qu'il s'agisse de silice pure ou de particules enrobées de silice hydrophile, doit être d'au moins 1 % en poids pour atteindre le but de l'invention, et elle peut aller par exemple, en poids de matière active, de 1 à 15 % en poids, mieux de 2 à 10 % en poids et encore mieux de 2 à 6 % en poids par rapport au poids total de la composition.

Les silices hydrophiles pouvant être utilisées dans la composition de l'invention sont de préférence amorphes et elles sont d'origine pyrogénée ou d'origine précipitée. Elles se présentent sous forme pulvérulente ou en dispersion aqueuse.

Les silices pyrogénées sont obtenues par pyrolyse en flamme continue à 1000°C du tétrachlorure de silicium ($SiCl_4$) en présence d'hydrogène et d'oxygène.

Les silices précipitées sont obtenues par réaction d'un acide sur des solutions de silicates alcalins, de préférence du silicate de soude.

On peut utiliser une silice ou un mélange de deux silices ou plus.

La silice hydrophile selon l'invention est choisie parmi les silices ayant une surface spécifique de 30 à 500 $m^2/g$, une dimension moyenne de particules en nombre allant de 3 à 50 nm et une densité tassée allant de 40 à 200 et mieux de 50 à 150 g/l. Ce sont plus particulièrement les silices hydrophiles décrites dans les tableaux (1) et (2) ci-dessous, et leurs mélanges.

Tableau(1)

| Nom commercial | AEROSIL 90 (Société Degussa-Hüls) | AEROSIL 130 (Société Degussa-Hüls) | AEROSIL 150 (Société Degussa-Hüls) | AEROSIL 200 (Société Degussa-Hüls) |
|---|---|---|---|---|
| Mode d'obtention | Pyrogénation | Pyrogénation | Pyrogénation | Pyrogénation |
| Surface BET ($m^2/g$) | 90± 15 | 130± 25 | 150± 15 | 200± 15 |
| Dimension moyenne des particules (nm) | 20 | 16 | 14 | 12 |
| Densité tassée (g/l) | Environ 80 | Environ 50 | Environ 50 | Environ 50 |
| Densité des groupes silanol ($OH/m^2$) | 2-3 | 2-3 | 2-3 | 2-3 |
| pH à 4% dans l'eau | 3,6-4,5 | 3,6-4,5 | 3,6-4,3 | 3,6-4,3 |
| Remarque | | | | taille des agrégats : 10-30 et 200 μ |

Tableau (2)

| Nom commercial | AEROSIL 300 (Société Degussa-Hüls) | AEROSIL 380 (Société Degussa-Hüls) | AEROSIL OX 50 (Société Degussa-Hüls) | SILICE FK 320 DS (Société Degussa-Hüls |
|---|---|---|---|---|
| Mode d'obtention | Pyrogénation | Pyrogénation | Pyrogénation | Précipitation |
| Surface BET ($m^2/g$) | 300± 30 | 380± 30 | 50± 25 | 170± 25 |
| Grandeur moyen des particules (nm) | 7 | 7 | 40 | 18 |
| Densité tassée (g/l) | Environ 50 | Environ 50 | Environ 130 | Environ 80 |
| Densité des groupes silanol ($OH/m^2$) | 2-3 | 2-3 | 2-3 | - |
| pH à 4% dans l'eau | 3,6-4,5 | 3,6-4,5 | 3,8-4,5 | 6,3 |

On peut aussi utiliser de la silice en dispersion aqueuse, et par exemple une dispersion de silice colloïdale, telle que le produit commercialisé sous la dénomination BINDZIL 30/220® par la société Eka Chemicals, dispersion colloïdale de silice amorphe (taille : 14 nanomètres) dans l'eau (30/70).

La silice hydrophile utilisée dans la composition de l'invention peut consister également en une particule recouverte totalement ou partiellement de silice, notamment d'une particule minérale recouverte totalement ou partiellement de silice, telle que les billes de silice contenant de l'oxyde de titane, commercialisées sous la dénomination TORAYCERAM S-IT® par la société Toray ; les microsphères de silice-alumine contenant de l'oxyde de titane (taille : 105 μ), commercialisées sous la dénomination Z-LIGHT-SPHERE W 1012® par la société Zeelan ; les particules de silice synthétique précipitée amorphe/oxyde de titane (taille : 106-500 μ), commercialisées sous la dénomination NEOSIL PC20S® par la société Crosfield ; les fibres de Nylon-6 - silice - oxyde de titane (longueur de 2 mm et épaisseur de 2 deniers), commercialisées sous la dénomination FIBERLON Y2® par la société Wackherr ; la silice enrobée de dioxyde de titane et recouvert de silice poreuse (85/5/10) (taille : 0,6 μ), commercialisée sous la dénomination ACS-0050510® par la société SACI-CFPA ; le nano-oxyde de titane anatase traité alumine et silice à 40% dans l'eau (taille : 60 nm,

monodisperse), commercialisé sous la dénomination MIRASUN TIW 60® par la société Rhodia Chimie CRA ; le nano-oxyde de titane anatase (60 nm) enrobé silice/alumine/cerium IV 15/5/3 en dispersion aqueuse à 32 %, commercialisé sous la dénomination MIRASUN TIW 160® par la société Rhodia Chimie CRA ; le nano-oxyde de titane anatase traité alumine et silice (34/4,3/1,7) en dispersion aqueuse à 40 %, commercialisé sous la dénomination TIOVEIL AQ-N® par la société Uniqema ; le nano-oxyde de titane enrobé de silice (66/33) (granulométrie du dioxyde de titane : 30 nm ; épaisseur de silice : 4 nm), commercialisé sous la dénomination MAXLIGHT TS-04® par la société Nichimen Europe PLC ; et le nano-oxyde de titane enrobé de silice (80/20) (granulométrie dioxyde de titane : 30 nm ; épaisseur de silice : 2 nm) commercialisé sous la dénomination MAXLIGHT TS-042® par la société Nichimen Europe PLC.

Ces particules peuvent également avoir des propriétés optiques dans le produit comme sur la peau. Par exemple, elles peuvent avoir un effet matifiant ou légèrement blanchissant.

On utilise de préférence comme silice hydrophile, les silices pyrogénées et en particulier celles commercialisées sous les dénominations AEROSIL 200® et AEROSIL 300® par la société Degussa-Hüls.

<u>Composés oxyalkylénés</u>

Le ou les composés oxyalkylénés qui peuvent être utilisés dans la composition peuvent comprendre des groupes d'oxyde d'éthylène (composés oxyéthylénés), des groupes d'oxyde de propylène (composés oxypropylénés) ou les deux (composés oxyéthylénés/oxypropylénés).

On peut utiliser un ou plusieurs composés oxyalkylénés, et la quantité de composé(s) oxyalkyléné(s) dans la composition de l'invention peut aller par exemple, en poids de matière active, de 1 à 20 % en poids, et mieux de 2 à 10 % en poids par rapport au poids total de la composition.

Les composés oxyalkylénés peuvent être choisis notamment parmi les polyéthylène glycols, les esters de polyéthylène glycol et/ou de polypropylène glycol, les éthers de polyéthylène glycol et/ou de polypropylène glycol, les dérivés acyl alkoxylés et notamment acyl éthoxylés de polyol, les triesters de glycérol et d'acides gras oxyalkylénés et notamment oxyéthylénés, les dérivés uréthane éthoxyéthylénés modifiés par des chaînes alkyle, et leurs mélanges.

1. Les polyéthylènes glycol pouvant être utilisés dans la composition sont des polycondensats d'oxyde d'éthylène, ayant un nombre de motifs d'oxyde d'éthylène (OE) supérieur à 10. Le nombre d'oxyde d'éthylène peut aller par exemple de 10 à 50.000 et de préférence de 14 à 10.000. Comme polyéthylène glycols, on peut citer par exemple le polyéthylène glycol comportant 7000 OE (nom CTFA : PEG-7M) comme le produit commercialisé sous la dénomination POLYOX WSR N-750® par la société Amerchol, le polyéthylène glycol comportant 75 OE (nom CTFA : PEG-75), le polyéthylène glycol comportant 20000 OE (nom CTFA: PEG-20M) comme le produit commercialisé sous la dénomination POLYOX WSR 1105® par la société Amerchol, le polyéthylène glycol comportant 150 OE (nom CTFA : PEG-150).

2. Les esters de polyéthylène glycol et/ou de polypropylène glycol sont des condensats de polyéthylène glycol et/ou polypropylène glycol avec un ou plusieurs acides gras. Ce sont des composés de formule :

$$RCOO - (OE)m - (OP)n - R'$$

dans laquelle $0 \leq m \leq 300$ et $0 \leq n \leq 300$ et $m+n \geq 6$, R et R' représentent indépendamment l'un de l'autre l'hydrogène ou une chaîne alkyle saturée ou insaturée, linéaire ou ramifiée, hydroxylée ou non, comportant de 1 à 30 atomes de carbone et de préférence de 12 à 22 atomes de carbone, ou une chaîne aryle, à condition que R et R' ne soit pas en même temps l'hydrogène.

Comme esters d'acide et de polyéthylène glycol et/ou de polypropylène glycol, on peut citer par exemple le distéarate de polyéthylène glycol (150 OE) tel que le produit commercialisé sous la dénomination ATLAS G-1821® par la société Uniqema, le PEG-150 dibehenate tel que le produit commercialisé sous la dénomination ETHOX PEG 6000 Dibehenate® par la société Ethox, le palmitostéarate de polyéthylène glycol (120 OE) tel que le produit commercialisé sous la dénomination STEARATE 6000 WL 1644® par la société Gattefosse, le copolymère de polyéthylène glycol (30 OE) et d'acide 12- hydroxystéarate tel que le produit commercialisé sous la dénomination ARLACEL P135® par la société Uniqema, le stéarate de polyéthylène glycol (40 OE) tel que le produit commercialisé sous la dénomination MYRJ 52® par la société Uniqema.

Dans le cas où R=R'=H, on peut citer par exemple le copolymère statistique polyoxyéthylène / polyoxypropylène (17 OE / 6 OP) commercialisé sous la référence UCON 75-H-450® par la Société AMERCHOL. Les molécules comportant plus d'OE et/ou plus d'OP ne sont pas exclues.

3. Les éthers de polyéthylène glycol et/ou de polypropylène glycol sont des condensats de polyéthylène glycol et/ou polypropylène glycol avec un ou plusieurs alcools gras. Ce sont des composés de formule :

R - (OE)m - (OP)n - R'

dans laquelle 0≤m≤300 et 0≤n≤300 et m+n ≥ 6, R et R' représentent indépendamment l'un de l'autre l'hydrogène ou une chaîne alkyle saturée ou insaturée, linéaire ou ramifiée, hydroxylée ou non, comportant de 1 à 30 atomes de carbone et de préférence de 12 à 22 atomes de carbone, ou une chaîne aryle, à condition que R et R' ne soit pas en même temps l'hydrogène.
Comme exemples d'éther de polyéthylène glycol, on peut citer notamment l'alcool cétylique oxyéthyléné (30 OE) tel que le produit commercialisé sous la dénomination NIKKOL BC-30TX t® par la société Nikkol, l'alcool oléylique oxyéthyléné (15 OE) tel que le produit commercialisé sous la dénomination NIKKOL BO-15TX® par la société Nikkol, l'alcool oléylique oxyéthyléné (50 OE) tel que le produit commercialisé sous la dénomination NIKKOL BO-50® par la société Nikkol, l'alcool béhénylique oxyéthyléné (10 OE) tel que le produit commercialisé sous la dénomination MERGITAL B 10® par la société Nikkol, l'alcool béhénylique oxyéthyléné (30 OE) tel que le produit commercialisé sous la dénomination NIKKOL BB-30® par la société Nikkol, l'alcool laurylique oxyéthyléné (12 OE) tel que le produit commercialisé sous la dénomination REWOPAL 12® par la société Goldschmidt, l'alcool laurylique oxyéthyléné (23 OE) tel que le produit commercialisé sous la dénomination SIMULSOL P 23® par la société Seppic, l'alcool octyl-2 dodécylique oxyéthyléné (20 OE) tel que le produit commercialisé sous la dénomination OCTYLDODECETH-20® par la société Stearinerie Dubois, l'alcool iso-cétylique oxyéthyléné (20 OE) tel que le produit commercialisé sous la dénomination ARLASOLVE 200 US® par la société Uniqema, l'alcool oléylique oxyéthyléné (10 OE) tel que le produit commercialisé sous la dénomination BRIJ 97 ® par la société Uniqema, l'alcool oléylique oxyéthyléné (20 OE) tel que le produit commercialisé sous la dénomination BRIJ 98® par la société Uniqema, l'alcool stéarylique oxyéthyléné (100 OE) tel que le produit commercialisé sous la dénomination BRIJ 700® par la société Uniqema, l'alcool stéarylique oxyéthyléné (21 OE) tel que le produit commercialisé sous la dénomination BRIJ 721® par la société Uniqema.
Comme exemples d'éther de polyéthylène glycol/polypropylène glycol, on peut citer notamment l'alcool laurylique oxyéthyléné (5 OE) oxypropyléné (5 OP) tel que le produit commercialisé sous la dénomination AETHOXAL B® par la société Cognis, l'alcool myristylique oxypropyléné (3 OP) tel que le produit commercialisé sous la dénomination PROMYRISTYL PM-3® par la société Croda, l'alcool cétylique oxyéthyléné (20 OE) oxypropyléné (5 OP) tel que le produit commercialisé sous la dénomination PROCETYL AWS® par la société Croda, l'alcool butylique oxyéthyléné (26 OE) oxypropyléné (26 OP) tel que le produit commercialisé sous la dénomination PPG-26-BUTETH-26® par la société Goldschmidt, l'alcool butylique oxyéthyléné (26 OE) oxypropyléné (26 OP) tel que le produit commercialisé sous la dénomination VARONIC APEB®) par la société Goldschmidt, le décyl-tetra-decanol oxyéthyléné (30 OE) oxypropyléné (6 OP) tel que le produit commercialisé sous la dénomination NIKKOL PEN-4630® par la société Nikkol, l'alcool laurylique oxyéthyléné (25 OE) oxypropyléné (25 OP) tel que le produit commercialisé sous la dénomination ADF-OLEILE®) par la société Vevy.

4. Les dérivés alkyl ou acyl éthoxylés de polyol utilisés dans la composition de l'invention sont des dérivés oxyéthylénés d'esters d'acide gras ou d'éthers d'alcool gras et de polyol tel que glycérol, sorbitol, glucose, pentaerythritol. Comme dérivés de ce type, on peut citer par exemple, le cocoate de glycéryle oxyéthyléné (78 OE) tel que le produit commercialisé sous la dénomination SIMULSOL CG par la société Seppic, le di-oléate de méthyl-glucose oxyéthyléné (120 OE) tel que le produit commercialisé sous la dénomination GLUCAMATE DOE-120 VEGETAL® par la société Amerchol, le septa-oléate de sorbitane oxyéthyléné (40 OE) tel que le produit commercialisé sous la dénomination ARLATONE T ® par la société Uniqema, le laurate de polyglycéryle (2 moles de glycérol) oxyéthyléné (10 OE) tel que le produit commercialisé sous la dénomination HOE S 3495 ® par la société Clariant, l'isostéarate de glycéryle oxyéthyléné (60 OE) tel que le produit commercialisé sous la dénomination EMALEX GWIS-160 ® par la société SACI-CFPA, le monostéarate de glycéryle oxyéthyléné (20 OE) tel que le produit commercialisé sous la dénomination CUTINA E 24 ® par la société Cognis, le stéarate de glycéryle oxyéthyléné (200 OE) tel que le produit commercialisé sous la dénomination SIMULSOL 220 TM ® par la société Seppic, le tétra-stéarate de pentaérythrityle oxyéthyléné (150 OE) tel que le produit commercialisé sous la dénomination CROTHIX ® par la société Croda.

5. Comme triesters de glycérol et d'acides gras oxyalkylénés, on peut citer par exemple les glycérides d'acide caprylique/caprique oxyéthylénés (6 OE), tels que le produit commercialisé sous la dénomination SOFTIGEN 767® par la société Condea, et l'huile d'olive oxyéthylénée (50 OE) tel que le produit commercialisé sous la dénomination CROVOL O-70® par la société Croda.

6. Comme dérivés uréthane éthoxyéthylénés modifiés par des chaînes alkyle, on peut citer par exemple ceux de formules (1) et (2) :

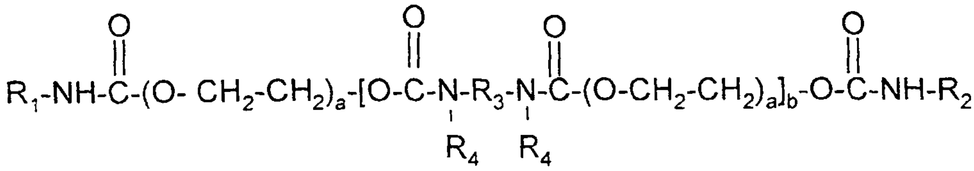

(1)

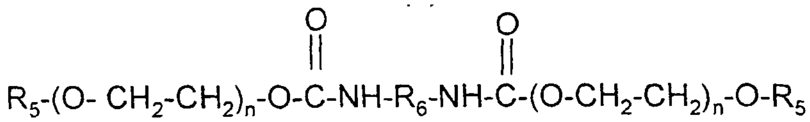

(2)

dans lesquelles les radicaux $R_1$, $R_2$ et $R_5$ représentent un groupe alkyle de $C_{1-18}$; $R_3$ et $R_6$ représentent un radical hydrocarboné linéaire, cyclique ou aromatique en $C_{4-36}$; $R_4$ représente un atome d'hydrogène ou un radical alkyle en $C_{1-6}$, de préférence un atome d'hydrogène ; a est un nombre entier allant de 90 à 600, et b est un nombre entier allant de 1 à 4.

Il s'agit par exemple de polymères hydrosolubles obtenus par réaction d'addition de diisocyanates (HMDI : Hexamethylene diisocyanate) sur des diols (polyether, polyesters) et terminés par des groupements hydrophobes provenant d'alcools gras éthoxylés ou éthoxylés/propoxylés. C'est le cas par exemple du SER AD FX 1100, commercialisé par la société ADRISS, qui est un copolymère alcool stéarylique oxyéthyléné (100 OE) / polyéthylène glycol (136 OE) /hexaméthylène diisocyanate.

Comme polymères de ce type, on peut citer par exemple les produits commercialisés sous les dénominations ACRYSOL 44 (ou ACULYN 44) et ACRYSOL 46 (ou ACULYN 46) (nom CTFA : PEG-150/DECYL ALCOHOL/SMDI COPOLYMER), qui sont des polyuréthannes obtenus par condensation d'hexaméthylène diisocyanate et de polyéthylèneglycol, portant à leurs extrémités respectivement un résidu méthyle et un résidu octadécyle. Ces polyuréthannes contiennent, en plus, de 3 à 5% d'une matrice d'amidon modifié par voie enzymatique. Ils sont commercialisés par la Société Rohm & Haas. Ce sont également les RHEOLATE® 205, 210, 212, 216, 244, 278, 255, 266, 288, 300, 350 commercialisés par la Société ELEMENTIS ou encore les BORCHIGEL LW.44, L.75.N; L 76; VP 9628-LL36; VP 97105-NT40 ; VP 9620 commercialisés par la Société BORCHERS.

Tensioactifs

La composition moussante selon l'invention contient au moins un tensioactif qui va apporter le caractère moussant à la composition. Ce tensioactif peut être choisi parmi tous les tensioactifs moussants non ioniques, anioniques, amphotères et zwitterioniques et leurs mélanges.

La quantité de tensioactif(s) peut aller par exemple, en poids de matière active, de 2 à 50 % en poids et mieux de 3 à 30 % en poids par rapport au poids total de la composition.

1. Les tensioactifs non ioniques :

On peut utiliser par exemple comme tensioactifs non ioniques, les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10 ® par la société Kao Chemicals, le produit commercialisé sous la dénomination PLANTAREN 2000 UP ® par la société Henkel, et le produit commercialisé sous la dénomination ORAMIX NS 10 ® par la société Seppic ; le caprylyl/capryl glucoside comme le produit commercialisé sous la dénomination ORAMIX CG 110 ® par la Société Seppic ; le laurylglucoside comme les produits commercialisés sous les dénominations PLANTAREN 1200 N ® et PLANTACARE 1200 ® par la société Henkel ; et le coco-glucoside comme le produit commercialisé sous la dénomination PLANTACARE 818/UP ® par la société Henkel.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit commercialisé sous la dénomination CHIMEXANE NF® par la société Chimex.

2. Les tensioactifs anioniques peuvent être choisis notamment parmi les carboxylates, les dérivés des aminoacides, les alkyl sulfates, les alkyl éther sulfates, les sulfonates, les iséthionates, les taurates, les sulfosuccinates, les alkyl sulfoacétates, les phosphates et alkylphosphates, les polypeptides, les dérivés anioniques d'alkyl polyglucoside, les savons d'acides gras, et leurs mélanges.

Comme carboxylates, on peut citer par exemple les sels alcalins de N-acylaminoacides ; les amido éthercarboxylates (AEC) comme le lauryl amido ether carboxylate de sodium (3 OE) commercialisé sous la dénomination AKYPO FOAM 30® par la société Kao Chemicals ; les sels d'acides carboxyliques polyoxyéthylénés, comme le lauryl ether carboxylate de sodium (C12-14-16 65/25/10) oxyéthyléné (6 OE) commercialisé sous la dénomination AKYPO SOFT 45 NV® par la société Kao Chemicals ; les acides gras d'huile d'olive polyoxyéthylénés et de carboxymethyl, produit commercialisé sous la dénomination OLIVEM 400® par la société Biologia E Tecnologia ; le tri-decyl éther carboxylate de sodium oxyéthyléné (6 OE) commercialisé sous la dénomination NIKKOL ECTD-6NEX ® par la société Nikkol.

Les dérivés des aminoacides peuvent être choisis par exemple parmi les sarcosinates et notamment les acylsarcosinates comme le lauroyl sarcosinate de sodium commercialisé sous la dénomination SARKOSYL NL 97® par la société Ciba ou commercialisé sous la dénomination ORAMIX L 30® par la société Seppic, le myristoyl sarcosinate de sodium, commercialisé sous la dénomination NIKKOL SARCOSINATE MN® par la société Nikkol, le palmitoyl sarcosinate de sodium, commercialisé sous la dénomination NIKKOL SARCOSINATE PN ® par la société Nikkol ; les alaninates comme le N-lauroyl-N-methylamidopropionate de sodium, commercialisé sous la dénomination SODIUM NIKKOL ALANINATE LN 30® par la société Nikkol ou commercialisé sous la dénomination ALANONE ALE®, par la société Kawaken, et le N-lauroyl N-methylalanine triéthanolamine, commercialisé sous la dénomination ALANONE ALTA ® par la société Kawaken ; les N-acylglutamates comme le mono-cocoylglutamate de triéthanolamine commercialisé sous la dénomination ACYLGLUTAMATE CT-12® par la société Ajinomoto, et le lauroyl-glutamate de triéthanolamine commercialisé sous la dénomination ACYLGLUTAMATE LT-12® par la société Ajinomoto ; les aspartates comme le mélange de N-lauroylaspartate de triéthanolamine et de N-myristoylaspartate de triéthanolamine, commercialisé sous la dénomination ASPARACK ® par la société Mitsubishi ; les citrates, et leurs mélanges.

Comme alkyl éther sulfates, on peut citer par exemple le lauryl éther sulfate de sodium (C12-14 70/30) (2,2 OE) commercialisé sous les dénominations SIPON AOS 225® ou TEXAPON N702 PATE® par la société Henkel, le lauryl éther sulfate d'ammonium (C12-14 70/30) (3 OE) commercialisé sous la dénomination SIPON LEA 370® par la société Henkel, l'alkyl (C12-C14) éther (9 OE) sulfate d'ammonium commercialisé sous la dénomination RHODAPEX AB/20® par la société Rhodia Chimie.

Comme sulfonates, on peut citer par exemple les alpha-oléfines sulfonates comme l'alpha-oléfine sulfonate de sodium (C14-16) commercialisé sous la dénomination BIO-TERGE AS-40® par la société Stepan, commercialisé sous les dénominations WITCONATE AOS PROTEGE® et SULFRAMINE AOS PH 12® par la société Witco ou commercialisé sous la dénomination BIO-TERGE AS-40 CG® par la société Stepan, l'oléfine sulfonate de sodium secondaire commercialisé sous la dénomination HOSTAPUR SAS 30® par la société Clariant ; les alkyl aryl sulfonates linéaires comme le xylène sulfonate de sodium commercialisé sous les dénominations MANROSOL SXS30®, MANROSOL SXS40®, MANROSOL SXS93® par la société Manro.

Comme iséthionates, on peut citer les acyliséthionates comme le cocoyliséthionate de sodium, tel que le produit commercialisé sous la dénomination JORDAPON Cl P® par la société Jordan.

Comme taurates, on peut citer le sel de sodium de méthyltaurate d'huile de palmiste commercialisé sous la dénomination HOSTAPON CT PATE® par la société Clariant ; les N-acyl N-méthyltaurates comme le N-cocoyl N-methyltaurate de sodium commercialisé sous la dénomination HOSTAPON LT-SF® par la société Clariant ou commercialisé sous la dénomination NIKKOL CMT-30-T® par la société Nikkol, le palmitoyl methyltaurate de sodium commercialisé sous la dénomination NIKKOL PMT® par la société Nikkol.

Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C12-C14, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Henkel, le mono-sulfosuccinate d'amide laurique oxyéthyléné (5 OE) commercialisé sous la dénomination LEBON A-5000® par la société Sanyo, le sel di-sodique de mono-sulfosuccinate de lauryl citrate oxyéthyléné (10 OE) commercialisé sous la dénomination REWOPOL SB CS 50® par la société Witco, le mono-sulfosuccinate de mono-éthanolamide ricinoléique commercialisé sous la dénomination REWODERM S 1333® par la société Witco.

Comme phosphates et alkylphosphates, on peut citer par exemple les monoalkylphosphates et les dialkyl phosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de diester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER® par la société Condea, le sel de potassium ou de triéthanolamine de mono-alkyl (C12-C13) phosphate commercialisé sous les références ARLATONE MAP 230K-40® et ARLATONE MAP 230T-60® par la société Uniqema, le lauryl phosphate de potassium commercialisé sous la dénomination DERMALCARE MAP XC-99/09® par la société Rhodia Chimie.

Les polypeptides sont obtenus par exemple par condensation d'une chaîne grasse sur les aminoacides de céréale et notamment du blé et de l'avoine. Comme polypeptides, on peut citer par exemple le sel de potassium de la lauroyl protéine de blé hydrolysée, commercialisé sous la dénomination AMINOFOAM W OR® par la société Croda, le sel de triéthanolamine de cocoyl protéine de soja hydrolysée, commercialisé sous la dénomination MAY-TEIN SY® par la société Maybrook, le sel de sodium des lauroyl amino-acides d'avoine, commercialisé sous la dénomination PROTEOL OAT® par la société Seppic, l'hydrolysat de collagène greffé sur l'acide gras de coprah, commercialisé sous la dénomination GELIDERM 3000® par la société Deutsche Gelatine, les protéines de soja acylées par des acides de coprah hydrogénés, commercialisées sous la dénomination PROTEOL VS 22® par la société Seppic.

Les dérivés anioniques d'alkyl-polyglucosides peuvent être notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET® par la société Cesalpinia, le sel di-sodique d'ester sulfosuccinique de cocoylpolyglucoside (1,4), commercialisé sous la dénomination ESSAI 512 MP® par la société Seppic, le sel de sodium d'ester citrique de cocoyl polyglucoside (1,4) commercialisé sous la dénomination EUCAROL AGE-EC® par la société Cesalpinia.

Les savons d'acide gras qui peuvent être utilisés comme tensioactifs anioniques sont des acides gras d'origine naturelle ou synthétique, salifiés par une base minérale ou organique. La chaîne grasse peut comporter de 6 à 22 atomes de carbone, de préférence de 8 à 18 atomes de carbone. La base minérale ou organique peut être choisie parmi les métaux alcalins ou alcalino-terreux, les aminoacides, et les aminoalcools. Comme sels, on peut utiliser par exemple les sels de sodium, de potassium, de magnésium, de triéthanolamine, de N-méthylglucamine, de lysine et d'arginine. Comme savons, on peut citer par exemple les sels de potassium ou de sodium des acides laurique, myristique, palmitique, stéarique (laurate, myristate, palmitate et stéarate de potassium ou de sodium), et leurs mélanges.

3. Les tensioactifs amphotères et zwitterioniques peuvent être choisis par exemple parmi les bétaïnes, les N-alkylamidobétaïnes et leurs dérivés, les dérivés de la glycine, les sultaïnes, les alkyl polyaminocarboxylates, les alkylamphoacétates et leurs mélanges.

Comme bétaïnes, on peut citer par exemple la cocobétaïne comme le produit commercialisé sous la dénomination DEHYTON AB-30® par la société Henkel, la laurylbétaïne comme le produit commercialisé sous la dénomination GENAGEN KB® par la société Clariant, la laurylbétaïne oxyethylénée (10 OE), comme le produit commercialisé sous la dénomination LAURYLETHER(10 OE)BETAINE® par la société Shin Nihon Rica, la stéarylbétaïne oxyéthylénée (10 OE) comme le produit commercialisé sous la dénomination STEARYLETHER(10 OE)BETAINE® par la société Shin Nihon Rica.

Parmi les N-alkylamidobétaines et leurs dérivés, on peut citer par exemple la cocamidopropyl bétaine commercialisée sous la dénomination LEBON 2000 HG® par la société Sanyo, ou commercialisée sous la dénomination EMPIGEN BB® par la société Albright & Wilson, la lauramidopropyl bétaïne commercialisée sous la dénomination REWOTERIC AMB12P® par la société Witco.

Comme dérivés de la glycine, on peut citer le N-cocoylglycinate de sodium commercialisé sous la dénomination AMILITE GCS-12® par la société Ajinomoto.

Comme sultaines, on peut citer le cocoyl-amidopropylhydroxy-sulfobetaine commercialisé sous la dénomination CROSULTAINE C-50® par la société Croda. Come alkyl polyaminocarboxylates (APAC), on peut citer le cocoylpolyaminocarboxylate de sodium, commercialisé sous la dénomination AMPHOLAK 7 CX/C®,et AMPHOLAK 7 CX® par la société Akzo Nobel, le stéaryl-polyamidocarboxylate de sodium commercialisé sous la dénomination AMPHOLAK 7 TX/C par la société Akzo Nobel, la carboxyméthyloléyl-polypropylamine de sodium, commercialisé sous la dénomination AMPHOLAK XO7/C® par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP® par la société Rhodia Chimie, et le N-cocoyl-N-hydroxyéthyl-N-carboxyméthyl-éthylènediamine N-sodique (nom CTFA : sodium cocamphoacetate).

Parmi les tensioactifs cités ci-dessus, selon un mode particulier de réalisation de l'invention, on utilise plus particulièrement comme tensioactifs anioniques, les acylsarcosinates, les alkyl éther sulfates oxyéthylénés, les N-acyl N-méthyltaurates, les N-acylglutamates, les acyliséthionates, les sulfosuccinates, les phosphates et alkylphosphates, les polypeptides, les savons ; comme tensioactifs amphotères et zwitterioniques, les bétaïnes, les alkylamphoacétates ; comme tensioactifs non ioniques, les alkylpolyglucosides, l'O-octanoyl-6'-D-maltose, l'O-dodecanoyl-6'-D-maltose, le dodecanediol polyglycérolé (3,5 moles de glycérol), l'éthyl-2 hexyl oxy-carbonyl N-methyl glucamine; et les mélanges de ces tensioactifs.

Le milieu aqueux de la composition selon l'invention peut contenir, outre l'eau, un ou plusieurs solvants choisis parmi les alcools inférieurs comportant de 1 à 6 atomes de carbone, tels que l'éthanol ; et les polyols. Comme polyols, on peut citer la glycérine ; les glycols comme le butylène glycol, l'isoprène glycol, le propylène glycol, les polyéthylène glycols tels que le PEG-8 ; le sorbitol ;les sucres tels que le glucose, le fructose, le maltose, le lactose, le sucrose ; et leurs mélanges. La quantité de solvant(s) dans la composition de l'invention peut aller par exemple de 0,5 à 30 % en poids et de préférence de 2 à 20 % en poids par rapport au poids total de la composition.

Les compositions selon l'invention ont une viscosité allant de préférence de 25 poises (2,5 Pa.s) à 700 poises (70 Pa.s) et plus particulièrement de 50 poises (5 Pa.s) à 300 poises (30 Pa.s), ces viscosités étant. mesurées au moyen de l'appareil Rheomat Mettler RM 180 à 25°C, cet appareil étant équipé d'un mobile différent selon les viscosités, par exemple d'un mobile 2 pour les gammes de viscosités inférieures à 7 poises, d'un mobile 3 pour les gammes de viscosités de 2 à 40 poises, et d'un mobile 4 pour les gammes de viscosités de 20 poises à 80 poises.

Les compositions de l'invention peuvent contenir des adjuvants habituellement utilisés dans le domaine cosmétique, et notamment ceux utilisés dans les produits de nettoyage. Comme adjuvants, on peut citer par exemple les parfums, les conservateurs, les séquestrants (EDTA), les pigments, les nacres, les charges minérales ou organiques, matifiantes, blanchissantes ou exfoliantes, les colorants solubles, les filtres solaires, les actifs cosmétiques ou dermatologiques tels que les vitamines hydrosolubles ou liposolubles, les antiseptiques, les antiséborrhéïques, les antimicrobiens tels que le peroxyde de benzoyle, l'acide salicylique, le triclosan, l'acide azélaïque, et aussi les azurants optiques, les polymères non ioniques tels que le polyvinylpyrrolidone (PVP), les polymères anioniques, les corps gras incompatibles avec le milieu aqueux, comme les huiles ou les cires. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Comme charges, on peut citer les charges minérales telles que le talc ou silicate de magnésium (granulométrie: 5 microns) commercialisé sous la dénomination LUZENAC 15 M00® par la société LUZENAC, le kaolin ou silicate d'aluminium comme par exemple celui commercialisé sous la dénomination KAOLIN SUPREME® par la société IMERYS, ou les charges organiques telles que l'amidon comme par exemple le produit commercialisé sous la dénomination AMIDON DE MAIS B ® par la société ROQUETTE, les microsphères de Nylon comme celles commercialisées sous la dénomination ORGASOL 2002 UD NAT COS® par la société ATOCHEM, les microsphères à base de copolymère de chlorure de vinylidene/Acrylonitrile/methacrylonitrile enfermant de l'isobutane, expansées comme celles commercialisées sous la dénomination EXPANCEL 551 DE® par la société EXPANCEL. On peut ajouter aussi à la composition de l'invention des fibres comme par exemple des fibres de nylon (POLYAMIDE 0.9 DTEX 0.3 MM commercialisé par les Etablissements PAUL BONTE, des fibres de cellulose ou « Rayonne » (RAYON FLOCK RCISE NOOO3 MO4® commercialisé par la société CLAREMONT FLOCK CORPORATION).

Les compositions selon l'invention peuvent constituer notamment des produits de nettoyage ou de démaquillage de la peau (corps, visage, yeux), du cuir chevelu et/ou des cheveux.

Un autre objet de l'invention consiste en l'utilisation cosmétique de la composition telle que définie ci-dessus, comme produits de nettoyage et/ou de démaquillage de la peau, des yeux, du cuir chevelu et/ou des cheveux.

Les compositions selon l'invention peuvent constituer aussi une composition pour traiter les peaux grasses et/ou désinfecter la peau et/ou le cuir chevelu, notamment quand elles contiennent un antibactérien. En particulier les actifs spécifiques de traitement des peaux grasses peuvent y être inclus, comme par exemple l'acide salicylique, l'acide azélaïque, le triclosan, le piroctone olamine, la niacinamide (vitamine PP).

Un autre objet de l'invention est l'utilisation de la composition telle que définie ci-dessus pour la préparation d'une composition destinée à traiter les peaux grasses et/ou désinfecter la peau et/ou le cuir chevelu.

Un autre objet de l'invention consiste en un procédé cosmétique de nettoyage de la peau, des yeux, du cuir chevelu et/ou des cheveux, caractérisé par le fait qu'on applique la composition de l'invention, sur la peau, sur les yeux, sur le cuir chevelu et/ou sur les cheveux, en présence d'eau, et qu'on élimine la mousse formée et les résidus de salissure par rinçage à l'eau.

Dans le cas du nettoyage du visage, la composition selon l'invention peut constituer un masque qui est rincé après un temps de pose de 1 à 3 minutes.

Les exemples qui suivent servent à illustrer l'invention sans toutefois présenter un caractère limitatif. Les

quantités indiquées sont en % en poids sauf mention contraire.

Le tableau (3) ci-dessous présente un exemple selon l'invention (composition moussante sans savon) et des exemples comparatifs. Les caractéristiques rhéologiques ont été obtenues au moyen du Rheomètre Haake RS150, à 25°C.

Tableau (3)

| Composition | Ex. comparatif 1 | Ex. comparatif 2 | Ex. comparatif 3 | Ex. 1 selon l'invention |
|---|---|---|---|---|
| Coco-bétaine [DEHYTON AB-30® à 30% en matière active (M.A.)] | 0 | 9,75% (M.A.) | 9,75% (M.A.) | 9,75% (M.A.) |
| Sodium laureth sulfate [TEXAPON N702 PATE® à 70% en matière active (M.A.)] | 0 | 2,6% (M.A.) | 2,6% (M.A.) | 2,6% (M.A.) |
| PEG-120 methyl glucose dioleate (GLUCAMATE DOE-120 VEGETAL®) | 4% | 0 | 4% | 4% |
| Silice hydrophile (AEROSIL 200®) | 5% | 5% | 0 | 5% |
| Sorbitol | 3,5% | 3,5% | 3,5% | 3,5% |
| Glycérine | 3,5% | 3,5% | 3,5% | 3,5% |
| Conservateurs | qs | qs | qs | qs |
| eau | Qsp100g | Qsp100g | Qsp100g | Qsp100g |
| Aspect | Produit liquide comme de l'eau (viscosité de 1 à 100 cPoises) | Produit translucide, liquide comme de l'eau (viscosité de 1 à 100 cPoises) | Gel cristallin coulant | Gel translucide épais. |
| pH | 6,6 | 6,7 | 7 | 6,2 |
| Viscosité sous cisaillement à 1000 $s^{-1}$ à environ 25°C | | 0,035 Pa.s | 0,0185 Pa.s | 0,032 Pa.s |
| Caractéristiques rhéologiques<br>- G* (Pa) à $10^{-2}$ Hz<br>- G* (Pa) à 1 Hz<br>- δ (deg.) à $10^{-2}$ Hz<br>- δ (deg.) à 1 Hz | | <br>0,01<br>0,6<br>88<br>90 | <br>0,16<br>17<br>90<br>89 | <br>564<br>958<br>11<br>23 |

Ainsi, seul l'exemple selon l'invention qui comprend un tensioactif moussant et de la silice hydrophile (Aerosil 200) associée à un composé oxyéthyléné (PEG-120 methyl glucose dioléate) possède le comportement de type « solide mou » recherché, avec δ variant de 2 à 45°, pour des sollicitations dont les fréquences vont de $10^{-2}$ à 1 Hz. Les valeurs de viscosité à 1000 $s^{-1}$ montrent que la composition de l'invention et les compositions des exemples comparatifs se comportent de façon similaire sous cisaillement, alors qu'elles se comportent différemment au repos : la composition de l'invention a une consistance épaisse au repos (exemple 1), ce qui n'est pas le cas des compositions des exemples comparatifs qui sont liquides (exemples comparatifs 1 et 2) ou coulant (exemple comparatif 3). Ceci montre bien le comportement de type « solide mou » et rhéofluidifiant de la composition de l'invention. Du fait qu'elle est épaisse au

repos, la composition de l'invention a l'avantage de permettre une meilleure préhension et une plus grande facilité d'application.

Performances sensorielles : les qualités de mousse développées sont évaluées selon le protocole décrit ci-dessous.

Avant toute utilisation des produits, les mains sont lavées au savon de Marseille puis convenablement rincées et séchées. Puis le protocole suivi est le suivant :

1- mouiller les mains en les passant sous l'eau courante, les secouer trois fois pour les essorer,
2- placer 1 g de produit dans le creux de l'une des mains,
3- travailler le produit entre les deux paumes pendant 10 secondes,
4- ajouter 2 ml d'eau et travailler le produit à nouveau pendant 10 secondes,
5- rincer les mains sous l'eau,
6- les essuyer.

Les critères sont évalués à chaque étape du protocole suivi, et ils sont notés sur une échelle de 0 à 10.

- étape 3 : évaluation du pouvoir couvrant : la note attribuée est d'autant plus élevée que la peau ne se voit pas à travers le produit étalé.
- étape 4 : évaluation de la qualité de mousse
- *Le volume de mousse :* la note attribuée est d'autant plus élevée que le volume est grand.
- *La taille des bulles composant la mousse :* la note attribuée est d'autant plus élevée que les bulles sont grosses.
- *La densité :* consistance, tenue de la mousse : la note attribuée est d'autant plus élevée que la densité est grande.
- *La douceur de la mousse :* la note attribuée est d'autant plus élevée que la mousse est douce.
- étape 5 : évaluation pendant le rinçage
- *Le rinçage :* la note attribuée est d'autant plus faible que la présence d'un film glissant difficile à éliminer est grande.

Les résultats sensoriels pour chacun des critères sont les suivants :

| | Ex. comparatif 3 | Ex. 1 selon l'invention |
|---|---|---|
| Vitesse d'apparition des premières bulles | 10 | 9,8 |
| Pouvoir couvrant | 6,3 | 6,4 |
| Volume de la mousse | 6,4 | 6,6 |
| Taille des bulles | 4,5 | 4,8 |
| Densité | 6,9 | 7,8 |
| Douceur de la mousse | 6,4 | 6,1 |
| Rinçage | 6,9 | 7,1 |

L'écart entre deux valeurs doit être supérieur ou égal à 1 pour être significatif.

Ainsi, l'addition de silice hydrophile et de composé oxyéthyléné ne modifie pas la vitesse d'apparition des premières bulles, malgré la rhéologie très modifiée de la composition selon l'invention par rapport au témoin.

Exemple 2 : Composition moussante sans savon

| | | |
|---|---|---|
| Mono-phosphate de lauryle (à 75% de mono-ester) (MAP 20®) | 6,5 % | (en M.A.) |
| Alkyl-C9/C11-polyglucoside (1.4) (MYDOL 10) | 6,5 % | (en M.A.) |
| Hydroxyde de potassium | 1,7% | |
| PEG-120 methyl glucose dioleate (GLUCAMATE DOE-120 VEGETAL®) | 2 % | |
| Silice hydrophile (Aerosil 200®) | 5 % | |
| Conservateurs | qs | |

(suite)

| | |
|---|---|
| Eau | Qsp 100 % |

On obtient un gel épais non coulant de pH 7,1, qui mousse bien en donnant une mousse douce et fine. ce gel a une viscosité sous cisaillement à 1000 $s^{-1}$ de 0,06 et les caractéristiques rhéologiques suivantes :

- G* (Pa) à $10^{-2}$ Hz 2170
- G* (Pa) à 1 Hz 2670
- $\delta$ (deg.) à $10^{-2}$ Hz 6
- $\delta$ (deg.) à 1 Hz 17

Exemple 3 : Composition moussante contenant des savons

| | |
|---|---|
| Laurate de potassium | 2 % |
| Myristate de potassium | 3,3 % |
| Palmitate de potassium | 2,3% |
| Stearate de potassium | 3,7 % |
| PEG-180 | 2 % |
| AEROSIL 200® | 3 % |
| Sorbitol | 3,5 % |
| Glycérine | 3,5 % |
| Butylène glycol | 6 % |
| Conservateurs | qs |
| Eau | Qsp 100 % |

La composition se présente sous forme d'un produit translucide épais de pH 9, ayant l'aspect d'une crème et donnant une mousse fine et douce.

**Revendications**

1. Composition de nettoyage comprenant dans un milieu aqueux physiologiquement acceptable comportant au moins 35 % en poids d'eau par rapport au poids total de la composition, (1) au moins un tensioactif moussant, (2) au moins 1 % en poids d'au moins une silice hydrophile par rapport au poids total de la composition, la silice hydrophile étant choisie parmi les silices d'origine pyrogénée ou d'origine précipitée, et la silice hydrophile ayant une surface spécifique de 30 à 500 $m^2/g$, une dimension moyenne de particules en nombre allant de 3 à 50 nm et une densité tassée allant de 40 à 200 g/l et (3) au moins un composé oxyalkyléné choisi parmi les dérivés alkyl ou acyl éthoxylés de polyol.

2. Composition selon la revendication 1, **caractérisée en ce qu'**elle a un module complexe G* allant de $10^2$ à $10^5$ Pa et un angle de perte $\delta$ allant de 2 à 45° pour des fréquences allant de 0,01 à 10 Hz.

3. Composition selon la revendication 1 ou 2, **caractérisée en ce qu'**elle comprend de 35 à 95 % en poids d'eau par rapport au poids total de la composition. '

4. Composition selon l'une quelconque des revendications précédentes, **caractérisée en ce que** la quantité de silice (s) hydrophile(s) va de 1 à 15 % de matière active en poids par rapport au poids total de la composition.

5. Composition selon l'une quelconque des revendications précédentes, **caractérisée en ce que** la silice hydrophile est une silice pyrogénée.

6. Composition selon l'une quelconque des revendications 1 à 4, **caractérisée en ce que** la silice hydrophile consiste en une particule enrobée de silice hydrophile.

7. Composition selon l'une quelconque des revendications précédentes, **caractérisée en ce que** la quantité de composé(s) oxyalkyléné(s) va de 1 à 20 % en poids de matière active, par rapport au poids total de la composition.

8. Composition selon l'une quelconque des revendications précédentes, **caractérisée en ce que** le tensioactif moussant est choisi parmi les tensioactifs non ioniques, les tensioactifs anioniques, les tensioactifs amphotères et zwitterioniques et leurs mélanges.

9. Composition selon l'une quelconque des revendications précédentes, **caractérisée en ce que** la quantité de tensioactif(s) moussant(s) va de 2 à 50 % en poids de matière active par rapport au poids total de la composition.

10. Composition selon l'une quelconque des revendications précédentes, **caractérisée en ce que** le tensioactif moussant est choisi parmi les alkylpolyglucosides, les esters de maltose, les alcools gras polyglycérolés, les dérivés de glucamine, les carboxylates, les dérivés des aminoacides, les alkyl sulfates, les alkyl éther sulfates, les sulfonates, les iséthionates, les taurates, les sulfosuccinates, les alkyl sulfoacétates, les phosphates et alkylphosphates, les polypeptides, les dérivés anioniques d'alkyl polyglucoside, les savons d'acides gras, les bétaïnes, les N-alkylamidobétaïnes et leurs dérivés, les dérivés de la glycine, les sultaïnes, les alkyl polyaminocarboxylates, les alkylamphoacétates et leurs mélanges.

11. Composition selon l'une quelconque des revendications précédentes, **caractérisée en ce qu'**elle comprend en outre au moins un solvant choisi parmi les alcools comportant de 1 à 6 atomes de carbone, les polyols, et leurs mélanges.

12. Utilisation cosmétique de la composition selon l'une quelconque des revendications 1 à 11, comme produits de nettoyage et/ou de démaquillage de la peau, des yeux, du cuir chevelu et/ou des cheveux.

13. Utilisation de la composition selon l'une quelconque des revendications 1 à 11, pour la préparation d'une composition destinée à traiter les peaux grasses et/ou désinfecter la peau et/ou le cuir chevelu.

14. Procédé cosmétique de nettoyage de la peau, des yeux, du cuir chevelu et/ou des cheveux, **caractérisé en ce qu'**on applique la composition selon l'une quelconque des revendications 1 à 11, sur la peau, sur les yeux, sur le cuir chevelu et/ou sur les cheveux, en présence d'eau, et qu'on élimine la mousse formée et les résidus de salissure par rinçage à l'eau.

15. Composition selon l'une quelconque des revendications 1 à 11, **caractérisée en ce qu'**elle constitue un masque.

**Claims**

1. Cleaning composition comprising, in a physiologically acceptable aqueous medium comprising at least 35% by weight of water relative to the total weight of the composition, (1) at least one foaming surfactant, (2) at least 1% by weight of at least one hydrophilic silica relative to the total weight of the composition, the hydrophilic silica being chosen from silicas of fumed origin or of precipitated origin, and the hydrophilic silica having a specific surface area of from 30 to 500 $m^2/g$, a numerical mean particle diameter ranging from 3 to 50 nm and a tamped density ranging from 40 to 200 g/l, and (3) at least one oxyalkylenated compound chosen from ethoxylated alkyl or acyl polyol derivatives.

2. Composition according to Claim 1, **characterized in that** it has a complex modulus G* ranging from $10^2$ to $10^5$ Pa and a loss angle $\delta$ ranging from 2° to 45° for frequencies ranging from 0.01 to 10 Hz.

3. Composition according to Claim 1 or 2, **characterized in that** it comprises from 35% to 95% by weight of water relative to the total weight of the composition.

4. Composition according to any one of the preceding claims, **characterized in that** the amount of hydrophilic silica (s) ranges from 1% to 15% on an active material weight basis relative to the total weight of the composition.

5. Composition according to any one of the preceding claims, **characterized in that** the hydrophilic silica is a fumed silica.

**6.** Composition according to any one of Claims 1 to 4, **characterized in that** the hydrophilic silica consists of a particle coated with hydrophilic silica.

**7.** Composition according to any one of the preceding claims, **characterized in that** the amount of oxyalkylenated compound(s) ranges from 1% to 20% on an active, material weight basis relative to the total weight of the composition.

**8.** Composition according to any one of the preceding claims, **characterized in that** the foaming surfactant is chosen from nonionic surfactants, anionic surfactants, amphoteric surfactants and zwitterionic surfactants, and mixtures thereof.

**9.** Composition according to any one of the preceding claims, **characterized in that** the amount of foaming surfactant (s) ranges from 2% to 50% on an active material weight basis relative to the total weight of the composition.

**10.** Composition according to any one of the preceding claims, **characterized in that** the foaming surfactant is chosen from alkyl polyglucosides, maltose, esters, polyglycerolated fatty alcohols, glucamine derivatives, carboxylates, amino acid derivatives, alkyl sulfates, alkyl ether sulfates, sulfonates, isethionates, taurates, sulfosuccinates, alkyl sulfoacetates, phosphates and alkyl phosphates, polypeptides, anionic alkyl polyglucoside derivatives, fatty acid soaps, betaines, N-alkylamidobetaines and derivatives thereof, glycine derivatives, sultaines, alkyl polyaminocarboxylates and alkylamphoacetates, and mixtures thereof.

**11.** Composition according to any one of the preceding claims, **characterized in that** it also comprises at least one solvent chosen from alcohols comprising from 1 to 6 carbon atoms and polyols, and mixtures thereof.

**12.** Cosmetic use of the composition according to any one of Claims 1 to 11, as a product for cleansing and/or for removing make-up from the skin, the eyes, the scalp and/or the hair.

**13.** Use of the composition according to any one of Claims 1 to 11, for the preparation of a composition intended for treating grease skin and/or for disinfecting the skin and/or the scalp.

**14.** Cosmetic process for cleansing the skin, the eyes, the scalp and/or the hair, **characterized in that** the composition according to any one of Claims 1 to 11 is applied to the skin, the eyes, the scalp and/or the hair, in the presence of water, and the lather formed and the soiling residues are removed by rinsing with water.

**15.** Composition according to any one of Claims 1 to 11, **characterized in that** it constitutes a mask.

## Patentansprüche

**1.** Reinigungszusammensetzung, umfassend in einem physiologisch unbedenklichen wässrigen Medium, das mindestens 35 Gew.-% Wasser in Bezug auf das Gesamtgewicht der Zusammensetzung umfasst, (1) mindestens ein schäumendes Tensid, (2) mindestens 1 Gew.-% an mindestens einer hydrophilen Kieselsäure in Bezug auf das Gesamtgewicht der Zusammensetzung, wobei die hydrophile Kieselsäure aus der Reihe der Kieselsäuren pyrogenen Ursprungs oder der Fällungskieselsäuren stammt und wobei die hydrophile Rieselsäure eine spezifische Oberfläche von 30-500 $m^2/g$, eine zahlenmittlere Teilchengröße von 3 bis 50 mm und eine Stampfdichte von 40 bis 200 g/l aufweist, und (3) mindestens eine Oxyalkylenverbindung aus der Reihe der Polyolethoxyacyl- oder -alkylderivate.

**2.** Zusammensetzung nach Anspruch 1, **dadurch gekennzeichnet, dass** sie bei Frequenzen von 0,01 bis 10 Hz einen komplexen Modul G* von $10^2$ bis $10^5$ Pa und einen Verlustwinkel $\delta$ von 2 bis 45° aufweist.

**3.** Zusammensetzung nach Anspruch 1 oder 2, **dadurch gekennzeichnet, dass** sie 35 bis 95 Gew.-% Wasser in Bezug auf das Gesamtgewicht der Zuaammensetzung aufweist.

**4.** Zusammensetzung nach einem der vorhergehenden Ansprüche, **dadurch gekennzeichnet, dass** die Menge an hydrophiler Kieselsäure bzw. hydrophilen Kieselsäuren 1 bis 15 Gew.-% Wirkstoff in Bezug auf das Gesamtgewicht der zuaammensetzung beträgt.

**5.** Zusammensetzung nach einem der vorhergehenden Ansprüche, **dadurch gekennzeichnet, dass** es sich bei der hydrophilen Kieselsäure um eine pyrogene Kieselsäure handelt.

6. Zusammensetzung nach einem der Ansprüche 1 bis 4, **dadurch gekennzeichnet, dass** die hydrophile Kieselsäure aus einem mit hydrophiler Kieselsäure beschichteten Teilchen besteht.

7. Zusammensetzung nach einem der vorhergehenden Ansprüche, **dadurch gekennzeichnet, dass** die Menge an oxyalkylenierter Verbindung bzw. oxyalkylenierten Verbindungen 1 bis 20 Gew.-% Wirkstoff in Bezug auf das Gesamtgewicht der Zusammensetzung beträgt.

8. Zusammensetzung nach einem der vorhergehenden Ansprüche, **dadurch gekennzeichnet, dass** das schäumende Tensid aus der Reihe der nichtionischen Tenside, der anionischen Tenside, der amphoteren Tenside, der zwitterionischen Tenside und ihren Mischungen ausgewählt ist.

9. Zusammensetzung nach einem der vorhergehenden Ansprüche, **dadurch gekennzeichnet, dass** die Menge an schäumenden Tensid bzw. schäumenden Tensiden 2 bis 50 Gew.-% Wirkstoff in Bezug auf das Gesamtgewicht der Zusammensetzung beträgt.

10. Zusammensetzung nach einem der vorhergehenden Ansprüche, **dadurch gekennzeichnet, dass** das schäumende Tensid aus der Reihe der Alkylpolyglucoside, Maltoseester, polyglycerinierten Fettalkohole, Glucaminderivate, Carboxylate, Aminosäurederivate, Alkylsulfate, Alkylethersulfate, Sulfonate, Isethionate, Taurate, Sulfosuccinate, Alkylsulfoacetate, Phosphate und Alkylphosphate, Polypeptide, anionischen Alkylpolyglucosidderivate, Fettsäureseifen, Betaine, N-Alkylamidobetaine und ihren Derivaten, Glycinderivate, Sultaine, Alkylpolyaminocarboxylate, Alkylamphoacetate und ihren Mischungen stammt.

11. Zusammensetzung nach einem der vorhergehenden Ansprüche, **dadurch gekennzeichnet, dass** sie weiterhin mindestens ein Lösungsmittel aus der Reihe der Alkohole mit 1 bis 6 Kohlenstoffatomen, Polyole und ihren Mischungen umfasst.

12. Kosmetische Verwendung der Zusammensetzung nach einem der Ansprüche 1 bis 11 als Reinigungs- und/oder Abschminkprodukte für die Haut, die Augen, die Kopfhaut und/oder das Haar.

13. Verwendung der Zusammensetzung nach einem der Ansprüche 1 bis 11 für die Herstellung einer Zusammensetzung zur Behandlung von fettiger Haut und/oder zum Desinfizieren der Haut und/oder der Kopfhaut.

14. Kosmetikverfahren zum Reinigen der Haut, der Augen, der Kopfhaut und/oder des Haars, **dadurch gekennzeichnet, dass** man die Zusammensetzung nach einem der Ansprüche 1 bis 11 in Gegenwart von Wasser auf die Haut, die Augen, die Kopfhaut und/oder das Haar aufbringt und den gebildeten Schaum und die Schmutzreste durch Spülen mit Wasser entfernt.

15. Zusammensetzung nach einem der Ansprüche 1 bis 11, **dadurch gekennzeichnet, dass** sie eine Maske darstellt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5880076 A
- EP 550281 A
- US 5389279 A
- JP 61180712 A
- WO 9628140 A
- EP 566438 A
- FR 2739556


### Littérature non-brevet citée dans la description


- **H.A. BARNES ; J.F. HUTTON ; K. WALTERS.** rheology. Elsevier, 1989, 46-54